# DEMANDE DE BREVET EUROPEEN

(11) **EP 1 183 999 A1**
(43) Date de publication de la demande: **06.03.2002**
(21) Numéro de dépôt: 00430028.1
(22) Date de dépôt: 04.09.2000
(51) Int. Cl.: A61B 17/17, A61F 2/36

(54) **Prothèse fémorale, et outillage de pose adapté à une telle prothèse**

(71) Demandeur: Prost, Didier, Dr., 13009 Marseille (FR); Cermolacce, Christophe, Dr., 13013 Marseille (FR)
(72) Inventeur: Prost, Didier, Dr., 13009 Marseille (FR); Cermolacce, Christophe, Dr., 13013 Marseille (FR)
(74) Mandataire: Rémont, Claude

(57) **Abrégé**

La présente invention est relative à une prothèse fémorale et à un outillage de pose spécialement adapté à une telle prothèse.

Le domaine technique de l'invention est celui de la fabrication de prothèses fémorales pour êtres humains.

Selon l'invention, une prothèse fémorale (1) comporte une collerette (2) saillante s'étendant entre la queue (3) et le col (4), et la collerette comporte une face (5) convexe d'appui sur la corticale (6).

## Description

La présente invention est relative à une prothèse fémorale et à un outillage de pose spécialement adapté à une telle prothèse.

Le domaine technique de l'invention est celui de la fabrication de prothèses fémorales pour êtres humains.

La demande de brevet FR 2.602.672 (MARQUER et al) décrit une prothèse fémorale comportant une partie supérieure constituée d'un col portant un cône morse apte à recevoir une rotule pour l'articulation de la prothèse du bassin, et une partie inférieure constituée d'une queue apte à être implantée dans la métaphyse et dans le canal médullaire du fémur ; afin que la prothèse puisse être implantée sans ciment dans le fémur, et soit facilement extractible, ce document décrit une prothèse comportant à la jonction du col et de la queue, une échancrure oblique inclinée de haut en bas vers le bord interne de la prothèse ; cette échancrure ayant la forme d'un fer à cheval s'étend sur la face antérieure, le bord interne et la face postérieure de la prothèse, sans concerner le bord externe de la prothèse ; cette échancrure reçoit par enfourchement une collerette oblique amovible de même forme que l'échancrure et d'épaisseur supérieure à la profondeur de celle-ci, des moyens étant prévus pour maintenir la collerette en place dans l'échancrure ; cette collerette saillante par rapport au col et à la queue de la prothèse sert de butée et permet l'appui de la prothèse sur la corticale interne du fémur après section de celui-ci et enfoncement de la queue dans le canal médullaire.

La coupe de la tête du fémur préalable à l'implantation d'une telle prothèse s'effectue généralement selon une première coupe droite sur laquelle peut prendre appui la collerette, et selon une deuxième coupe droite inclinée par rapport à la première et rejoignant celle-ci, cette deuxième coupe permettant de détacher la tête fémorale ; ces deux traits de coupe (non alignés) créent un point de fragilité à leur jonction, d'autant plus que la plupart des chirurgiens utilisent pour ce faire une scie oscillante, cette fragilisation pouvant conduire à une fracture du grand trochanter.

Un objectif de l'invention est de proposer une prothèse fémorale améliorée et qui remédie à cet inconvénient.

Un autre objectif est de proposer un outillage amélioré pour la pose d'une telle prothèse.

A cet effet, une prothèse fémorale selon l'invention comporte une collerette comportant une face de contact (et/ou d'appui) sur l'os cortical fémoral qui est, en partie au moins, de forme convexe, de préférence en forme de portion continue de calotte cylindrique dont la convexité est dirigée vers la queue.

Selon un autre aspect, l'invention consiste en un outillage (ou ancillaire) de coupe de la tête fémorale qui comporte un moyen de coupe selon un profil courbe (concave) dont la concavité est orientée vers la tête du fémur, et de préférence selon un profil en arc de cercle, de rayon sensiblement égal au rayon de courbure de la face d'appui de la collerette.

Des résultats particulièrement bons sont obtenus en choisissant un rayon de courbure (moyen) de la face convexe de la collerette et de la face concave de la découpe cylindrique, dont la valeur soit située dans une plage allant de 20 à 40 mm, en particulier de 25 à 35 mm.

La forme convexe de la face d'appui de la collerette et la forme concave de la coupe corticale permettent d'obtenir un appui correct de la collerette sur l'os, tout en facilitant un positionnement précis de l'implant de façon centrée dans le canal médullaire ; en outre, ces formes courbes conjuguées facilitent la mise sous pression du ciment péri-prothétique en évitant la fuite du ciment (liquide ou pâteux) par l'interstice entre la collerette et l'os, à la fin de la mise en place de la prothèse, c'est à dire dans les derniers millimètres de la course d'enfoncement de la prothèse ; ces formes assurent un contact amélioré qui se traduit par un interstice minime ou inexistant entre l'os et la collerette ; ceci permet d'éviter la migration vers l'interface de liaison os/prothèse de particules (de métal ou de polyéthylène notamment) résultant de l'usure des surfaces de frottement de la prothèse, et diminue par conséquent les risques de réactions biologiques susceptibles de provoquer un descellement de la tige fémorale.

Selon d'autres caractéristiques préférées de l'invention :
- la collerette est constituée par une pièce en forme de fourche, qui peut être séparable du corps (queue et col) de la prothèse ;
- l'outillage comporte une scie cloche équipée d'un doigt de centrage et de rayon sensiblement égal à celui de la face d'appui de la collerette.

Selon un autre aspect, l'invention consiste à proposer un outil de pose comportant un moyen pour percer dans la tête fémorale un trou (ou « avant-trou » ou orifice) apte à recevoir un doigt de centrage d'un outil de coupe apte à réaliser une coupe curviligne, en particulier un doigt de centrage d'une scie cloche.

Selon une caractéristique préférée de l'invention, l'outil (ou ancillaire) de pose comporte un gabarit de perçage du trou de centrage qui comporte un moyen d'appui du gabarit sur le grand trochanter, qui comporte un canon de perçage pour le guidage d'un outil de perçage, et qui comporte une structure déformable de liaison du moyen d'appui au canon de perçage, permettant la réalisation précise et facile d'un trou de guidage pour l'outil de coupe utilisé ultérieurement.

L'utilisation des outils de repérage et d'appui sur le grand trochanter, de perçage et de coupe circulaire, permettent une section nette et propre du col fémoral, sans zone de fragilité, directement reproductible et pouvant servir de zone de référence pour la descente de la tige de prothèse fémorale sans ciment, mais également pour la descente et la mise en place de la tige d'une prothèse fémorale cimentée.

Ainsi, selon un dernier aspect, l'invention consiste à proposer un ancillaire de mise en place d'une tige fémorale d'une prothèse totale de hanche qui se distingue des ancillaires connus par son point de référence situé sensiblement au sommet du grand trochanter fémoral.

Lors de la mise en place d'une tige fémorale d'une prothèse totale de hanche, avec les dispositifs connus, une grande difficulté existe en effet au niveau de la reproductibilité et de la réalisation technique du geste opératoire, par rapport à la planification préopératoire ; il est en effet difficile de prévoir exactement le futur emplacement de la tige fémorale dans la cavité médullaire du fémur ; cela est cependant très important puisque le centre de rotation de la prothèse de hanche doit idéalement se superposer au centre de rotation de la hanche naturelle ; or la position du centre de rotation de la prothèse est directement dépendante du niveau et de l'orientation de l'implant dans la cavité fémorale.

Les techniques utilisant comme repère le petit trochanter sont peu fiables, car le repère se situe le plus souvent au point de jonction proximal du petit trochanter à la diaphyse fémorale ; ce point est difficile à repérer en préopératoire car il est masqué par les attaches musculaires, et est de forme peu repérable, l'angle de raccordement du petit trochanter sur la diaphyse fémorale étant aigu.

L'invention consiste donc à proposer un ancillaire de coupe du col fémoral, un ancillaire de râpe du fût médullaire et un ancillaire de mise en place de l'implant définitif, qui sont dotés d'un moyen de repérage en un point situé au voisinage du sommet du grand trochanter.

Ce point peut facilement être déterminé sur une radiographie péropératoire de face de la hanche à opérer ; il est situé au point de contact entre le sommet osseux du grand trochanter et la perpendiculaire abaissée à la droite marquant l'axe du fémur.

Ce point est également facile à déterminer en préopératoire ; il permet de régler précisément le centre de rotation de l'ancillaire de coupe circulaire du col fémoral, la descente des râpes successivement utilisées pour la préparation du fût fémoral destiné à recevoir la queue (ou tige) de l'implant, la descente de l'implant définitif et par là-même d'être sûr du niveau et de la position du centre de rotation de la prothèse après sa mise en place.

Son repérage en péropératoire est assuré par un guide centromédullaire introduit dans la cavité médullaire fémorale au niveau de la fossette digitale du fémur et par une pièce coulissante de forme cylindrique qui descend jusqu'au contact du grand trochanter ; ce système d'appui sur le grand trochanter d'un guide muni de repères permet de régler la position du centre de rotation du système de coupe cylindrique du col fémoral.

Les avantages procurés par l'invention seront mieux compris au travers de la description suivante qui se réfère aux dessins annexés, qui illustrent sans aucun caractère limitatif des modes préférentiels de réalisation de l'invention.

Dans les dessins, les éléments identiques ou similaires portent, sauf indication contraire, les mêmes références d'une figure à l'autre.

La figure 1 illustre schématiquement en vue en coupe longitudinale un fémur équipé d'une prothèse ; la figure 2 illustre en coupe schématique une collerette intégrée à la prothèse, et est une vue selon II-II de la figure 1.

La figure 3 illustre un gabarit de perçage d'une tête fémorale pour la réalisation d'un trou de guidage d'une scie cloche représentée figure 5 ; la figure 4 illustre sous un autre angle, la partie du gabarit de la figure 3 qui sert de liaison réglable entre la tige du gabarit et le canon de perçage du gabarit.

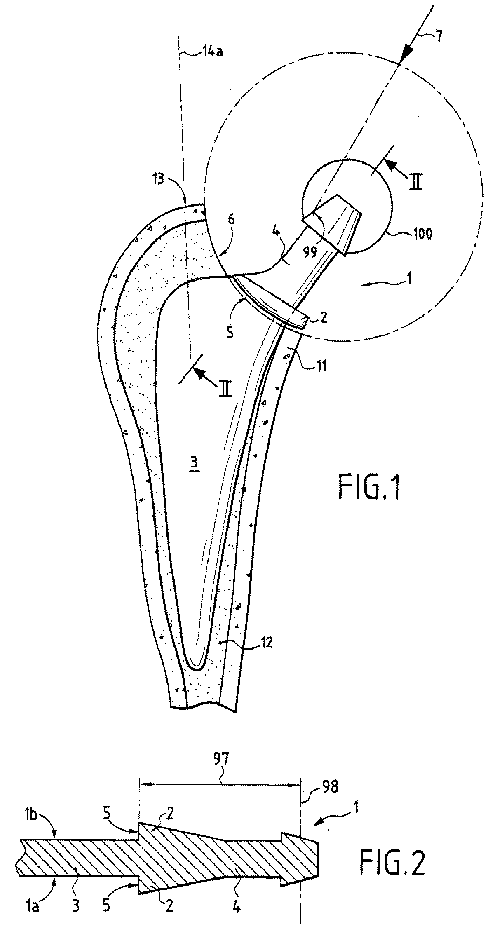

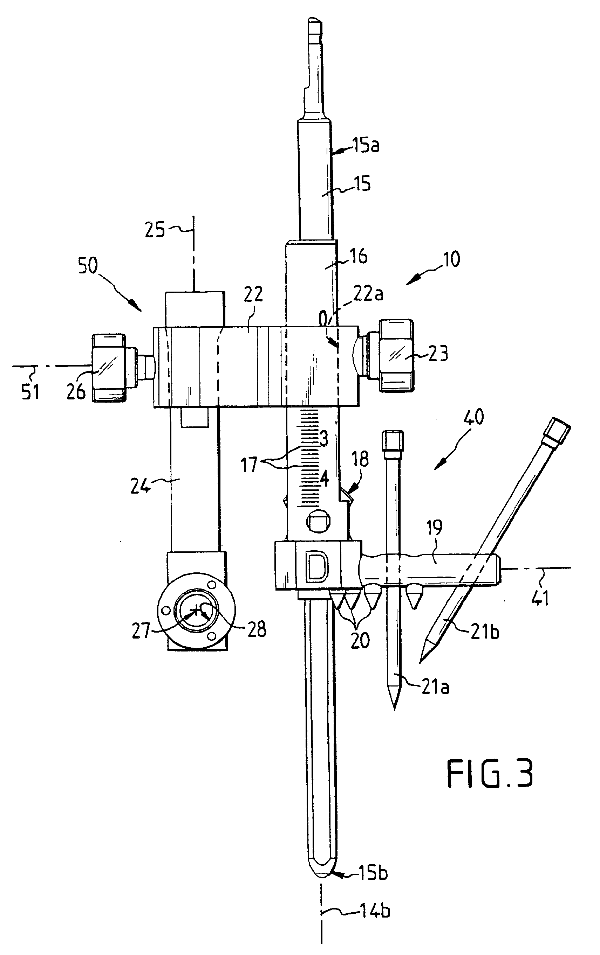

La prothèse 1 comporte (figures 1 et 2) un corps constitué d'une partie inférieure (queue) 3 et d'une partie supérieure (col) 4 dont l'extrémité est destinée à recevoir une tête prothétique 100 ; la prothèse est logée dans le canal médullaire 12 du fémur dont la tête a été séparée par une coupe (cylindrique) de centre 99 et de rayon 7, à l'aide d'une scie cloche ; de ce fait l'os cortical 11 se termine par une face 6 épousant le profil circulaire de la coupe réalisée ; la collerette 2 de la prothèse est dotée, la long de la face avant 1a et le long de la face arrière 1b de la prothèse, de deux faces 5 convexes, de forme cylindrique d'axe 98 et de rayon 97 égal au rayon 7 de la coupe réalisée ; ainsi la prothèse s'appuie par ses faces 5 sur l'extrémité 6 de l'os 11, sans qu'il subsiste d'interstice notable.

Afin de réaliser la coupe 6 cylindrique, la scie cloche 8 (figure 5) comporte - de façon habituelle - un doigt de centrage 9 allongé selon un axe 32 et prolongeant un embout 33 ; cet embout carré permet l'entraînement en rotation de la scie 8 selon l'axe 32 par un actionneur motorisé ou autre ; sur le doigt 9 est montée la lame 37 pourvue de dents 38 et en forme de cylindre creux entourant le doigt 9 et coaxial à celui-ci ; cette lame est fixée au doigt par l'intermédiaire d'un disque 36 solidaire d'un manchon 34 percé d'un trou taraudé 35 pour une vis de fixation ; pour le guidage de la lame 37 pendant la coupe de la tête fémorale, l'extrémité 9a du doigt 9, qui est saillante en avant des dents 38 d'une distance 9c, est introduite et maintenue dans un orifice cylindrique (d'axe 99) préalablement percé dans la tête fémorale, et dont le diamètre correspond à celui 9b du doigt 9 de la scie.

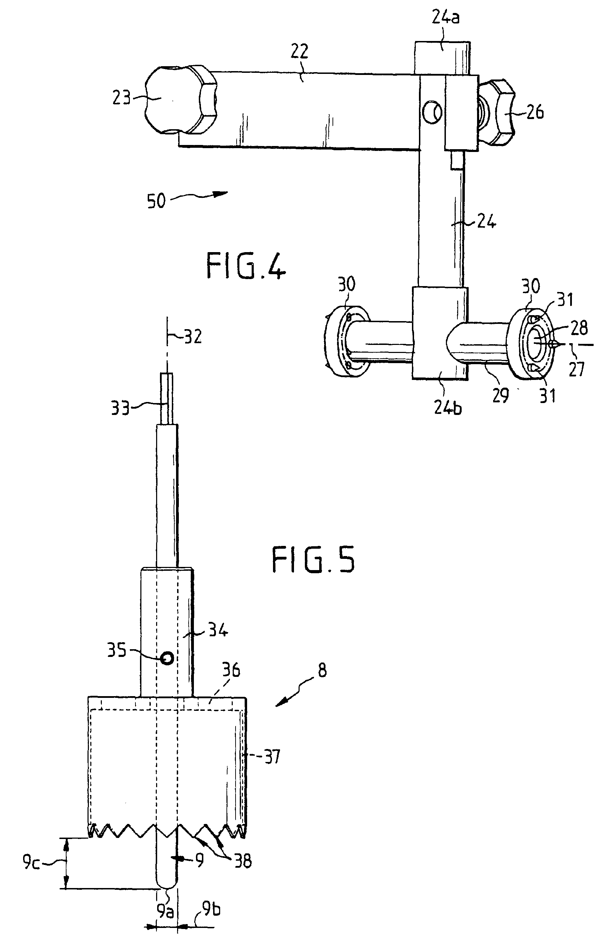

Afin de percer ce trou de guidage, le gabarit (figures 3, 4) permet de guider l'outil (mèche) de perçage grâce à un canon cylindrique 28, dont l'axe 27 doit être positionné de façon confondue avec l'axe 99 de coupe.

A cet effet, le gabarit permet la fixation temporaire du canon 28 au fémur, et le réglage de la position du canon pour correspondre à une position préalablement déterminée sur une radiographie, par référence au sommet 13 du grand trochanter.

La fixation du gabarit 10 au fémur est obtenue par l'ancrage de la tige 15 d'axe 14b dans l'os selon l'axe 14a s'étendant au voisinage du sommet 13 ; après enfoncement de la partie inférieure 15b, une structure 40 d'appui et de repérage est solidarisée à la tige 15 par un écrou 18 ; cette structure 40 est essentiellement constituée d'un tube 16 d'axe 14b gradué en 17 extérieurement, dont la base est solidaire d'une patte 19 transversale (d'axe 41) ; cette patte est munie sur sa face inférieure de dents (ou picots) 20 par lesquels la structure 40 s'appuie sur le grand trochanter ; un clou 21a parallèle à l'axe de la tige 15 et un clou 21b oblique peuvent être utilisés pour stabiliser la fixation temporaire du gabarit 10 dans l'os ; une structure 50 de support du canon 28, qui permet son réglage et son blocage en position par référence aux dents 20 en contact avec le grand trochanter, comporte un bras 22 d'axe 51 transversal, qui est muni d'un alésage 22a permettant son coulissement et son pivotement autour du tube 16 ; une vis 23 permet le blocage en position, à une hauteur et selon un angle déterminés, du bras 22 par rapport au tube 16 et à la tige 15.

Le bras 22 porte un arbre 24 d'axe 25 parallèle à l'axe 14b, à l'extrémité inférieure 24b duquel est fixé un tube 29 creux formant le canon 28 de perçage ; le tube 29 est réversible et muni de deux brides 30 d'extrémité pourvues de dents 31 permettant un appui stable sur la tête fémorale pendant le perçage.

La tige 15, le guide (16, 17) gradué, la pièce d'appui (19) et le canon (28) sont séparables et solidarisés par des vis (18, 23, 26).

## Revendications

1. Ancillaire de pose d'une prothèse fémorale (1) **caractérisé en ce qu'**il comporte un gabarit (10) de perçage dans la tête fémorale d'un orifice apte à recevoir un centreur d'un outil (8) de coupe curviligne, en particulier un doigt (9) de centrage d'une scie cloche, et **en ce que** ledit gabarit comporte un moyen (19, 20) d'appui sur le grand trochanter.

2. Ancillaire selon la revendication 1 dans lequel ledit gabarit (10) comporte :
- un canon (28) de perçage pour le guidage d'un outil de perçage dudit orifice, et
- une structure (40, 50) déformable de liaison du moyen d'appui (19, 20) au canon (28) de perçage.

3. Ancillaire selon la revendication 1 ou 2, dans lequel le gabarit comporte un guide (16, 17) apte à coulisser le long d'une tige (15) centromédullaire et une pièce (19, 20) dentée d'appui sur le grand trochanter.

4. Ancillaire selon la revendication 3, dans lequel le guide (16, 17) est gradué et qui comporte un organe (18) de blocage du guide (16, 17) en position sur la tige (15).

5. Ancillaire selon la revendication 3 ou 4, dans lequel le gabarit comporte un support (50) pivotant et coulissant qui relie le canon (28) de perçage à la tige (15).

6. Ancillaire selon l'une quelconque des revendications 1 à 5, qui comporte en outre des moyens (21a, 21b) de fixation temporaire du gabarit au fémur, tels que des clous.

7. Ancillaire selon l'une quelconque des revendications 3 à 6, dans lequel la tige (15), le guide (16, 17) gradué, la pièce d'appui (19) et le canon (28) sont séparables et solidarisés par des vis (18, 23, 26).

8. Ancillaire selon l'une quelconque des revendications 1 à 7, qui comporte en outre ledit outil (8) de coupe de la tête fémorale selon un profil courbe, qui est une scie cloche (8) équipée dudit doigt (9) de centrage.

9. Ancillaire selon la revendication 8, dans lequel le rayon de la scie cloche (8) est situé dans une plage allant de 20 mm à 40 mm.

10. Prothèse fémorale (1) dont la pose est destinée à être réalisée avec un ancillaire selon l'une quelconque des revendications 1 à 8, **caractérisée en ce qu'**elle comporte une collerette (2) saillante s'étendant entre la queue (3) et le col (4) et comporte une face (5) convexe d'appui sur la corticale (6), laquelle face (5) s'étend continuement selon une forme cylindrique, et dans laquelle le rayon (7) de courbure de la face (5) est situé dans une plage allant de 20 mm à 40 mm.

11. Prothèse selon la revendication 10, dans laquelle la collerette est en forme de fourche et est séparable du corps de la prothèse.
